# EUROPEAN PATENT APPLICATION

(11) **EP 2 716 298 A1**
(43) Date of publication of application: **09.04.2014**
(21) Application number: 12306208.5
(22) Date of filing: 03.10.2012
(51) Int. Cl.: A61K 38/05, G01N 33/50, C12N 5/074

(54) **A nod2-dependant pathway of cytoprotection of stem cells**

(71) Applicant: INSTITUT PASTEUR, 75724 Paris Cedex 15 (FR)
(72) Inventor: Sansonetti, Philippe J., 75014 Paris (FR); Nigro, Giulia, 75015 Paris (FR)
(74) Representative: Sellin, Carole

(57) **Abstract**

The present invention is directed to an agonist of NOD2 for use in therapy for increasing the autonomous capacity of survival of vertebrate adult stem cells, without loss of their capacity to multiply and differentiate, and preferably the capacity of survival of intestinal stem cells, especially in response to a stress. The invention also concerns the use of an agonist of Nod2 for increasing *in vitro* or ex *vivo* the autonomous capacity of survival, without loss of multiplication and differentiation capacity of mammalian adult stem cell. The invention also discloses different media and support for mammalian adult stem cells. The invention also concerns an *in vitro* screening process for identifying molecules capable increasing, in response to a stress, the autonomous capacity of survival, without loss of multiplication and differentiation capacity of mammalian adult stem cells.

## Description

The present invention is concerned with mammalian stem cells, and more specifically the cytoprotection of this type of cells.

Stem cells are unspecialized cells that have two defining properties, namely the ability to self-generate and the ability to differentiate into multiple cell types. Whereas embryonic stem cells can differentiate into all of the hundreds cell types of the body (totipotency or pluripotency) and are apparently immortal in culture, adult stem cells can differentiate into a limited range of adult cells (multi-potency) and they are mortal, although they have an extensive replication potential (Tower, 2012). Adult stem cells are found in variety of tissues, including intestine, brain, bone marrow, pancreas, liver, skin, skeletal muscle and kidney.

In different pathologies, some tissues or systems, like the skin or the hematopoietic system, may be damaged, non-functional or incapable of regeneration, as a result of dysfunctions at the stem cell level. Likewise, stem cells may be damaged by therapy, especially chemotherapy and radiotherapy applied for treating cancers, adversely affecting their survival and regeneration capacities and *in fine* jeopardizing the whole capacity of self-renewal of the tissue.

In view of their multi-potency and their extensive and sustained tissue renewal capacity, adult stem cells have been widely studied for the last decades.

The use of adult stem cells in research and therapy is not as the use of embryonic stem cells, because the production of adult stem cells does not require the destruction of an embryo.

Their transplantation with a view to reconstituting a damaged or absent tissue is practiced with promising results. As an illustration, Stelzner and Chen have been able to make new intestinal mucosa by transplanting mucosal stem cells from one rat donor to another one (Stelzner and Chen, 2006). Transplantation of hematopoietic stem cells to reconstitute the immune system, especially in case of radiotherapy or myeloablation, is also practiced in humans. Adult stem cell treatments have indeed been successfully used for many years to treat leukemia and related bone/blood cancers through bone marrow transplants. Adult stem cells are also used in veterinary medicine to treat tendon and ligament injuries in horses.

In order to obtain the best tissue or system repair results after transplantation of stem cells, it is however imperative that the stem cells be harvested and transplanted in favorable conditions, ensuring the best survival rate, without loss of self-renewal and differentiation potential, which is however a challenging objective, in view of the stressful conditions imposed by the cell manipulation consecutive to transplantation.

In addition to the above, stem cells are subjected to many other stresses *in vivo,* that is extrinsic or intrinsic stress. Stress reduction in stem cells is critical, due to their essential role in tissue renewal and because of the risk of malignant transformation.

Management of stress by adult stem cells is still poorly understood for the time being and there is thus a need for means for favoring stem cell survival when subjected to stress without lost of their self renewal and differentiation capacities.

The present inventors have shown that adult stem cells express the NOD2 protein, and that activation of this intracellular receptor in adult stem cells ensures a cytoprotection of these cells.

Nucleotide oligomerization domain (NOD) 2/Caspase activation and recruitment domain (CARD) 15 belongs to the described family of intracellular NOD-like receptor proteins (NLRs), which contain a central nucleotide-binding site domain flanked on its *C*-terminal side by a leucine-rich repeat domain and on its *N*-terminal side by two CARD domains. The NOD2 protein is known to play an important role in immune system function. The NOD2 protein is indeed expressed and active in some types of immune system cells, including monocytes and macrophages, and in dendritic cells. The protein is also active in several types of epithelial cells, including Paneth cells, which are found in the lining of the intestine. These cells help defend the intestinal wall against bacterial infection.

The NOD2 protein has several critical recognized functions in immune defense against foreign invaders. The protein is a cytosolic sensor for a conserved bacterially derived structure, namely Muramyl di-peptide (N-acetylmuramyl-L-Alanyl-D-Isoglutamine or MDP) and is capable of activating in response proinflammatory signaling pathways, such as the NF-κB pathway. This protein complex regulates also the activity of multiple other genes, involved in control immune responses and inflammatory reactions.

The critical importance of the NLR family of receptors in inflammatory processes is further reinforced by the characterization of Nod2 and Nalp3 as susceptibility genes for different inflammatory disorders. The most prominent example is the linkage between a frameshift mutation in the *Nod2* gene and the early onset of Crohn's disease.

Expression of *Nod2* in mesenchymal stem cells of human umbilical cord blood has been recently reported with an hypothetic role in induction of some differentiation pathways (Kim et al, 2010).

Unexpectedly, the present inventors have shown that an agonist of NOD2, namely MDP, is able to increase the number of organoids formed from isolated intestinal stem cells, and that this increase is NOD2-dependent, i.e. requires the presence of NOD2. Moreover, they have shown that, at the level of the intestinal crypts the nod2 gene is expressed not only by Paneth cells but also by stem cells in a higher level than in Paneth cells. They finally demonstrated that MDP has a cytoprotective effect on stem cells, via the NOD2-pathway, especially in stress conditions, where MDP is able to increase the survival capacity of stem cells and also to increase the capacity of said cells to regenerate tissue.

The present invention is thus generally directed to different uses, methods and compositions, based on the cytoprotectant effect of NOD2-agonist on adult stem cells, and more specifically vertebrate adult stem cells, preferably in mammalian adult stem cells.

In a first aspect, the invention concerns an agonist of NOD2 for increasing the autonomous capacity of survival, multiplication and differentiation of mammalian adult stem cells, namely for use in therapy.

By NOD2 is meant the wild-type Nucleotide Oligomerization Domain (NOD) 2 protein of the mammal in question, also called Caspase Activation and Recruitment Domain (CARD) 15. This protein is indeed highly conserved in different mammalian species. The corresponding sequences can be found in the protein database for different mammalian species or can be easily found by homology with the already known sequences for the other species. The sequence of human NOD2 protein is set forth in accession number AAG33677.1 (GI:11275614), and in SEQ ID N°1. The murine NOD2 sequence is set forth in SEQ ID N°2, corresponding to Genbank accession number AAN52478.1 (GI:30017199).

The increase in the capacity of survival, multiplication and differentiation is particularly relevant in stressful conditions, i.e. in conditions imposing a stress on the stem cells such that their survival, or their capacity to multiply and differentiate, is adversely affected. Indeed, when the stress imposed on the stem cells is such that said cells die or become unable to multiply and differentiate, their capacity of tissue regeneration is lost and they thus become useless for the organism. The present invention brings a protection to these stem cells, preventing at least in part, these changes imposed by the stressful conditions, and thus protecting the stem cells from dying or from becoming unable to multiply and differentiate. The agonist of NOD2 according to the present invention has thus a cytoprotectant effect on the stem cells, preventing their death and their loss of multiplication and differentiation capacity.

According to the invention, by mammalian adult stem cells is meant any mammalian cell fulfilling the functional definition: a cell that has the potential to regenerate a plurality of tissues over a lifetime, and which is not an embryonic stem cell, i.e. which is not derived from the epiblast tissue of the inner cell mass (ICM) of a blastocyst or earlier morula stage embryos. A stem cell can also be defined as a multipotent cell with a high self renewal capacity, with a telomerase activity and capable of becoming quiescent; wherein multipotent or multipotential means capable of differentiating into at least two cell types.

Preferably, a mammalian adult stem cell according to the present invention is not a mesenchymal stem cell derived from Human umbilical cord blood, which is an immature stem cell and not an adult stem cell.

The present inventors have indeed observed that the novel cytoprotection effect on adult stem cells, does not appear to be obtained on embryonic stem cells.

In the context of the present invention, the term "cytoprotectant effect" or "cytoprotection" of stem cells is thus to be understood as an increase in survival rate or viability of stem cells, accompanied by maintenance of their capacity of multiplication and differentiation, which means the maintenance of their potential of reconstituting a whole tissue. Stem cells protected according to the invention thus remain multi-potent.

It is stressed that the cytoprotection provided by an agonist of NOD2 according to the invention preferably applies at the single cell level, i.e. adult stem cells have individually an increased probability of remaining viable and being still capable of multiplication and differentiation.

In this respect, it must be noted that the cytoprotection provided by an agonist of NOD2 according to the invention is to be distinguished from a direct induction of differentiation. Indeed, the maintenance of the differentiation potential is different from an induction of commitment in differentiation pathways with loss of multipotency potential.

Cytoprotection, or increase in autonomous capacity of survival, multiplication and differentiation, provided by NOD2-agonist according to the invention, means that the treated adult stem cells are maintained alive and that, concomitantly, their capacity to self-renew and to replenish adult tissue is preserved.

By self-renewal, it is to be understood the capacity for division without altering the initial characteristics of the cell.

Moreover, according to the invention, the cytoprotection provided to stem cells by agonists of NOD2 is linked to an increase of the stem cells' own capacity to overcome stressful conditions, and not to the effect of further cells or factors to reduce stress.

The mammalian stem cells according to the invention may be any type of adult stem cells. Preferred types of adult stem cells are intestinal stem cells, mesenchymal stem cells, hematopoietic stem cells, skin stem cells and muscle stem cells. Other types of stem cells also envisaged by the present invention are adipose-derived stem cell, endothelial stem cell. Particularly preferred stem cells are NOD2 expressing stem cells. According to a particularly preferred embodiment of the invention, the adult stem cells are intestinal stem cells.

With regard to the stressful conditions imposed on the stem cells according to the invention; they relate *inter alia* to any conditions which do not correspond to the optimal conditions for normal growth of the stem cells. Stress or stressful conditions include conditions leading to loss of progenitor capacity and conditions leading to cell death.

Stress can indeed take several forms, at the level of the cell and at the level of the organism, it can be extrinsic or intrinsic, or both, like aging. Extrinsic environmental stresses include radiation (for example UV-radiation of X-ray radiation) and xenobiotic toxins, like anticancer therapies. Biomechanical stresses and physical stress are also encompassed by the present invention. They comprise shear stress and hydrodynamic stress that can damage the stem cells during manipulation.

According to preferred embodiments of the present invention, the stress is a chemical stress, particularly an oxidative stress, irradiation, or a pharmacological stress. A chemical stress can be defined *inter alia* by imposing to the stem cells a particular chemical environment that induces stress reactions and eventually loss of progenitor capacity and/or cell death. An example of a chemical stress is creation of conditions of oxidative stress in the cellular milieu/environment. An oxidative stress can be defined as exposure of cells to reactive oxygen species (ROS) produced by dedicated enzymatic complexes by the cells of interest or neighbouring cells under threat. Oxidative stress can be mimicked by addition of molecules like H₂O₂. Irradiation is preferably by UV or X-rays. A pharmacological stress can be defined by the exposure to drugs like anti-cancer drugs, particularly Doxorubicine that is particularly active on adult stem cells and is able, as an intercalating agent to induce DNA breaks causing induction of cell death pathways.

According to another embodiment of the present invention, the stress applied to the stem cells is a physical or physiopathological stress. By physical stress, it is understood physical conditions leading to a stress, for example a shear stress or hydrodynamic stress to the cell wall, likely to damage the cell. Said physical stresses may especially be experienced during manipulation of stem cells, either when harvested or extracted from a donor or re-implanted or re-infused into a recipient, or during any step of culture of said stem, including sampling, sorting, seeding, dilution, change of medium, change of support, etc....

A physiopathological stress can be defined as disturbances of bodily function resulting from diseases. Examples of physiopathological stresses likely to be applied to stem cells are *inter alia* infections, particularly bacterial and viral infections, or a stress generated by auto-immune response.

It is to be noted that both physical and pharmacological conditions tested also cause the generation of ROS.

Another type of stress may be linked to deficiencies of blood supply of the stem cells, linked to a defined pathology or to a stroke.

According to preferred embodiments of the present invention, the stress applied to the stem cells is induced by chemotherapy, by radiotherapy, by infection or by tissue injury or dissection.

According to other preferred embodiments, the stress is applied during any measures undertaken with a view to transplanting said stem cells, especially any measures taken before or during their harvest, extraction or collection, and any measures taken before, during or after their re-implantation or re-infusion. The transplantation may be autologous or non-autologous transplantation. In case of non-autologous transplantation, the stress applied to the stem cells may also be due to the immune response of the organism in which they are implanted, or to the drugs administered for diminishing this immune response.

A particularly preferred embodiment of the present invention lies in the use of an agonist of NOD2 specifically during transplantation of intestinal stem cells. Said transplantation of intestinal stem cells may indeed be carried out for treating intestinal atrophy or crypt dysfunction, or any other pathology leading to a disruption of the intestinal homeostasis. The intestinal atrophy may be linked to immunopathological diseases such as celiac diseases; it may also be linked to pediatric malnutrition in developing countries, in relation to epithelial atrophy as part of the syndrome of environmental enteropathy. In the frame of transplantation of organoids for treating Inflammatory Bowel Diseases, comprising intestinal stem cells, is also likely to be treated by an agonist of NOD2 according to the invention. According to another embodiment of the invention, the stem cells are skin stem cells and the treatment with an agonist of NOD2 is during transplantation of said stem cells. The transplantation of skin stem cells may indeed be carried out for treating any skin or other epithelial wounds, in order to accelerate skin or epithelial regeneration.

According to a further embodiment of the invention, the stem cells are hematopoiectic stem cells and the treatment with an agonist of NOD2 is during bone marrow transplantation.

According to another embodiment of the present invention, an agonist of NOD2 is for increasing *in situ* the autonomous capacity of survival, multiplication and differentiation of mammalian intestinal stem cells. This cytoprotection may be useful in particular for intestinal epithelial repair in inflammatory bowel diseases, in chemotherapies, in immunopathological diseases inducing intestinal atrophy or in pediatric malnutrition in relation to epithelial atrophy. In this cases indeed, administration of an agonist of NOD2 is likely to preserve the intestinal stem cells from the stress applied by the recited pathologies or therapies, thus increasing their survival rate and consequently their capacity to repair the intestine tissue or repopulate the crypt with stem cells.

The present invention is also directed to an agonist of NOD2 for use in transplantation of mammalian adult stem cells, for improving engraftment of said cells and tissue regeneration. Indeed, as detailed above, transplantation corresponds to highly stressful conditions for the stem cells, likely to induce their death or incapacity to proliferate and differentiate.

For this embodiment also, the stem cells to be transplanted are preferably NOD2-expression stem cells. Particularly preferred adult stem cells are *inter alia* adult intestinal, hematopoietic, mesenchymal or skin cells, as mentioned above. The different pathologies or diseases to be treated by transplantation of mammalian adult stem cells have been detailed above.

For the different therapeutic applications described, the agonist of NOD2 is preferably administered before or during the application of the stressful conditions. It may advantageously be applied (for example contacted with the stem cells) in the days before initiation of the stress. For example, in case of harvest or extraction of stem cells with a view to their transplantation, the agonist of NOD2 is preferably applied beginning at least two days before the day of harvest or extraction. In this case, it is advantageously applied regularly, once or several times per day until the intervention. The agonist may also be applied just before the stress, or only concomitantly with the stressful conditions.

According to the invention, it is also envisaged that the agonist of NOD2 be first applied after the beginning of the stressful conditions, and even after the return to optimal or normal conditions. In this case, the purpose of the NOD2 agonist is to increase the recovery rate or recovery capacity of the stem cells.

With regard to the regimen and dosage, it will be defined by the physician, taking account of the patient and the conditions endangering the stem cells.

By agonist of NOD2 (or "NOD2 agonist"), it is to be understood a molecule capable of interacting with the NOD2 receptor intracellularly and capable of triggering a NOD2-dependant pathway. A NOD2 dependent pathway may be the cytoprotectant effect according to the invention, it may also be activation of the NF-κB pathway or activation of the MAPK pathway. Preferably an agonist of NOD2 according to the invention is a compound capable of reproducing the cytoprotective effect on intestinal stem cells reported in the experimental section below with N-acetylmuramyl-L-Alanyl-D-Isoglutamine (Muramyl di-peptide). A suitable system for testing whether a compound is an agonist of NOD2 is for example the system described in Girardin et al. (EMBO Repts, 2001), wherein activation of NOD2 is followed by activation of the NF-kB pathway of signaling. Another suitable system corresponds to the experimental work detailed in the Example section below, using the number of organoids formed from intestinal stem cells for determining and assaying the cytoprotection provided by various compounds.

The well known natural agonist of NOD2 is MDP (Muramyl dipeptide), more specifically N-acetylmuramyl-L-Alanyl-D-Isoglutamine, having the formula (I) below. MDP is the minimal bioactive peptidoglycan (PGN) motif common to bacteria, either Gram+ or Gram- bacteria. The recognition between MDP and NOD2 is highly stereospecific of the L-D isomer, excluding any reaction to the D-D or L-L analogs. MDP can be chemically synthesized or can be extracted from bacterial PGN.

Peptidoglycan itself is also to be considered as an agonist of NOD2, through its capacity to release MDP. Any other compound capable of releasing MDP in conditions suitable for stem cells growth, is also to be considered as a NOD2 agonist. Prodrugs, releasing MDP in vivo or in conditions close to in vivo conditions, are encompassed in the definition of NOD2 agonist according to the present invention.

The present invention also encompassed any derivative of MDP, also capable of activating the NOD2 receptor. Such derivatives of MDP can be *inter alia* constituent of peptidoglycan of certain bacteria, differing slightly from the structure of MDP, however maintaining the same or improved capacity to activate NOD2.

An example of derivative of MDP is represented by formula (Ia) and any salts thereof, wherein:
R₁ represents H, CH₃ or an alkyl, aryl, arylalkyl, heteroaryl, heteroarylalkyl, cycloalkyl, heterocycloalkyl, alkoxy, heteroalkoxy, acyl or alkylamine moiety, having preferably between 1 to 24 carbon atoms, preferably between 1 to 12, preferably 1, 2, 3 or 4 carbon atoms. Preferably, R₁ is H, CH₃, OH, CH₂-OH, NH₃ or phenyl.
R₂ represents H, or an alkyl, aryl, arylalkyl, acyl, heteroaryl, heteroarylalkyl, cycloalkyl, heterocycloalkyl, alkoxy, heteroalkoxy, alkylamine or O-acyl moiety, having preferably between 1 to 24 carbon atoms, preferably between 1 to 10 carbon atoms. Preferably, R₂ is H, COOH, CO-NH₂ or CO-R, with R being a C₁₀-C₂₀ alkyl, aryl, arylalkyl, heteroaryl or heteroarylalkyl moiety.
Alkyl, aryl, arylalkyl, heteroaryl, heteroarylalkyl, cycloalkyl, heterocycloalkyl, alkoxy, heteroalkoxy, alkylamine and O-acyl moieties have their usual definitions and possess preferably less than 200 atoms or less than 100 atoms, even preferably less than 60 atoms, or less than 40 atoms.

A particularly preferred derivative is N-Glycolyl- Muramyl dipeptide, formula (II), which can be found in mycobacteria and differs from MDP only by the addition of N-Glycolyl moiety (R₁ is CH₂-OH).

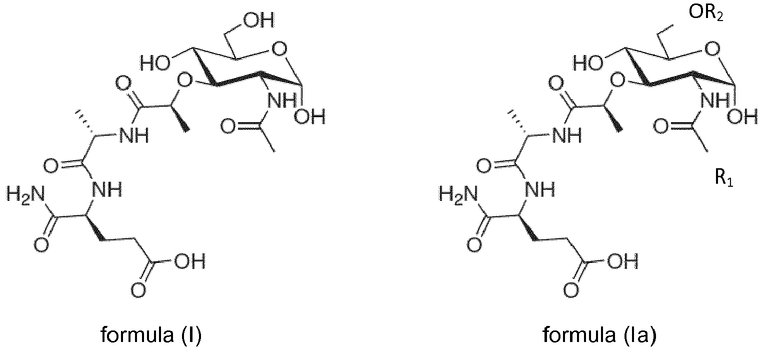

Example of another derivative is for example 6-O-acyl derivatives of Muramyl dipeptide, especially 6-O-stearoyl-N-Acetyl- muramyl-L-Alanyl-D-Isoglutamine according to formula (III) below.

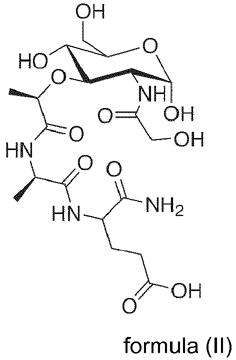

Other derivatives are also encompassed by the present invention, namely derivatives by addition of different moieties, likely to improve the transport of the molecule intracellularly, or to stabilize the molecule, or to limit any immune response.

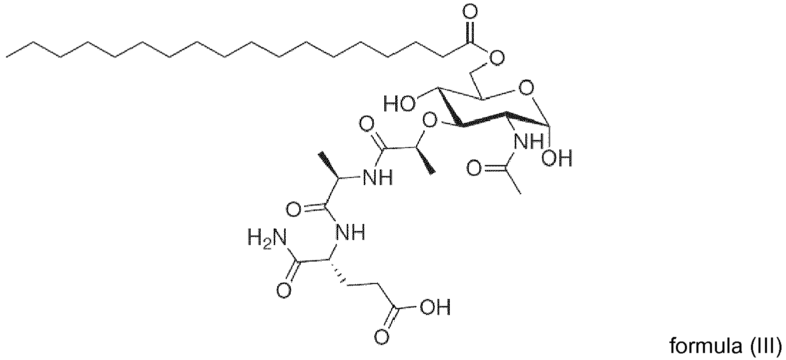

Other commercially available derivatives include the Muramyldipeptide with a C18 fatty acid chain, MurNAc-Ala-D-isoGln-Lys, naturally released by Lactobacillus salivarus, MurNAc-Ala-D-isoGln-L-Lys(D-Asn), naturally released by *Lactobacillus acidophilus* (Macho Fernandez et al., 2011), and murabutide (N-Acetyl-muramyl-L- Alanyl-D-Glutamin-n-butyl-ester). Other derivatives can be envisaged by the skilled person in the art without specific difficulties. Particularly preferred agonists of NOD2 are molecules capable of passing through the cell membrane, either naturally or through endogenous transporters, NOD2 being an intracellular receptor.

The NOD2 agonist according to the invention may be administered by any appropriate means, for example by injection or by oral route, under any appropriate form. It can be administered in association with one or several pharmacologically acceptable carriers. It can also be associated with any other active substances; it may also be administered separately, concomitantly or sequentially with other active principles. It is also envisaged according to the present invention to combine two different NOD2 agonists.

In a preferred embodiment of the present invention, the mammalian stem cells to be treated by the agonist of NOD2 are human stem cells. The therapeutic applications described above are indeed well suited to treatment of human beings. Alternatively, the mammalian stem cells may also be rat or murine stem cells, porcine stem cells, simian stem cells, or any other type of stem cells likely to represent a suitable model system for humans. According to a further embodiment of the invention, the stem cells may be from cat, dog, horse, pets or cattle.

It must be noted in this respect that, although the sequences of NOD2 receptors from different mammals are slightly different, it is expected that MDP is able to activate all these different orthologs, insofar as MDP is a natural molecule and the minimal bioactive peptidoglycan motif common to all bacteria and thus naturally present in the gut of any mammals.

According to a second aspect, the present invention is more specifically directed to different uses of agonists of NOD2, especially *in vitro* or *ex vivo* uses of said agonists, during culture or any type of manipulation of vertebrate adult stem cells, preferably mammalian adult stem cells. Indeed, in view of the ability of agonists of NOD2 as detailed with respect to the first aspect of the invention, an agonist of NOD2 can be used according to the invention for increasing *in vitro* or *ex vivo* the autonomous capacity of survival, multiplication and differentiation of mammalian adult stem cells.

In case of *in vitro* or *ex vivo* culture of mammalian adult stem cells, these stem cells can advantageously be stimulated by an agonist of NOD2, in order to increase the viability of these cells and to ensure that they do not lose their capacity to multiply and differentiate during the culture.

This *in vitro* or *ex vivo* use according to the invention can advantageously be carried out on an adult stem cell population, preferably on an homogeneous population, i.e. containing only adult stem cells, more particularly, on a clonal population; this use can also be carried out on tissue explants of blood samples for example, or on any tissue explants comprising adult stem cells.

The step of culture is a key step in all fundamental uses and studies on stem cells, or example studies on their role and function. It is indeed important that the stem cells not become senescent (i.e not dying) during this culture step, especially because stem cells are difficult to obtain, at least for primary human stem cells, and because an important phenomenon studied on these cells is aging, which necessitates very long culture periods and thus requires that the stem cells remain viable and maintain their self-renewal capacity. The step of *in vitro* culture is also critical in the course of transplantation, after harvest or extraction and before re-implantation or re-infusion.

Addition of agonist of NOD2 is particularly preferred when the stem cells are experiencing a stress, when they are recovering from a stress, or in preparation for application of a stress. The present inventors have indeed shown that, by activation of the NOD2 pathways, through treatment with an agonist of NOD2, stem cells are then protected against stressful conditions, especially their mortality rate is less than the mortality rate of those stem cells which are not treated with the agonist, and they keep their capacity to multiply and differentiate at a better rate than untreated cells. Activation of the NOD2 pathway can advantageously be made in the hours preceding the stress, during the stress, or even in the hours following the stress, in order to improve recovery of the stem cells.

For this aspect of the invention, the type of adult stem cells is the same as for the first aspect of the invention. The adult stem cells are thus preferably adult intestinal stem cells, mesenchymal stem cells, hematopoietic stem cells, muscle stem cells or skin stem cells. Any other mammalian adult stem cells can also be envisaged.

The stem cells likely to be treated by the agonist of NOD2 according to the invention are NOD2 expressing stem cells.

The populations of stem cells to be treated by an agonist of NOD2 according to the invention are preferably entirely homogeneous, i.e. they contain only stem cells. More particularly, the populations may be clonal populations.

Particularly preferred stem cells are intestinal stem cells. They have been shown recently capable of forming *in vitro* organoids (Sato et al, 2009). Intestinal stem cells or organoids can be transplanted into a recipient, in order to reconstitute a functional mucosa. Transplantation of organoids can indeed be carried out to treat different Inflammatory Bowel Diseases. It is thus imperative that, during culture, the intestinal stem cells remain viable and capable of differentiation, or that the number of organoids formed from harvested stem cells be as high as possible.

As detailed in the experimental section below, the present inventors have shown that the formation of organoids is greatly enhanced in presence of an agonist of NOD2. Indeed, the number of organoids formed, when the intestinal stem cells are stimulated by an agonist of NOD2, is greater than the number of organoids formed in the absence of said agonist, and the number of dying cells is less, when the stem cells are stimulated with an agonist of NOD2. Moreover, after stimulation by NOD2, there are still stem cells having the capacity to differentiate in all the lineages of the tissue, i.e for intestinal stem cells, in all the four major differentiated cell types: enterocytes, goblet cells, enteroendocrine cells and Paneth cells. The stress applied to these stem cells can be any type of stress. Numerous potential stresses have been detailed with regard to the first aspect of the invention and are also applicable to this aspect of the invention, where the stem cells are cultured *in vitro* or *ex vivo.* Namely, the stress applied to the stem cells can be *inter alia* a chemical stress, an oxidative stress, a pharmacological stress, a physical stress or an infectious stress.

*In vitro* or *ex vivo,* the stress can be induced *inter alia* by manipulation, culture, sampling or isolation of said adult stem cells.

Examples of stresses more specific to the *in vitro* or *ex vivo* culture are the stress applied to the culture during change of media, the stress applied by sampling or sorting. Moreover, specific manipulations can be carried out on the stem cells, likely to induce an important stress. Namely, during *in vitro* or *ex vivo* culture of stem cells, it is possible to transduce or transfect these stem cells, e.g. for inserting a foreign resistance gene, or to repair a defective gene. In case of gene therapy for example, during the culture of the stem cells obtained from a patient, these cells are transduced to correct the defective gene or to insert the lacking gene, such that the "corrected" stem cells are then re-implanted or re-infused into the patient. The protection of adult stem cells provided by the agonist of NOD2 according to the invention is recommended in any situation requiring their purification and re-implantation, which are by essence stressful conditions.

Agonists of NOD2 are particularly well adapted during these manipulation steps, which are damaging the stem cells and generally lead to the death of numerous stem cells.

Moreover, insofar as these stem cells are intended for re-implantation or re-infusion, it is of utmost importance that the cells not only survive, but also keep their potential to regenerate the whole tissue, i.e. keep their capacity to multiply and differentiate into a plurality of tissues. Indeed, stress or stressful conditions include both conditions leading to loss of progenitor capacity and conditions leading to cell death.

Potential agonists of NOD2 have been detailed with respect to the first aspect of the invention. These molecules are also suitable for this second aspect of the invention. Namely, a particularly preferred agonist is the Muramyl-dipeptide (N-acetylmuramyl-L-Alanyl-D-Isoglutamine), which is a constituent of any bacteria.

Also envisaged according to the present invention are any functional derivatives of MDP, especially derivatives by addition of a functional moiety. This added moiety may increase the capacity to get into the stem cells, to stabilize the compound, or for example to render it compatible with any other components of the cell culture medium.

Examples of potential derivatives are those detailed above, especially compounds of formula (Ia), and preferably N-Glycolyl- Muramyl dipeptide of formula (II) or a 6-O-acyl derivative of Muramyl dipeptide, especially 6-O-stearoyl-N-Acetyl- muramyl-L-Alanyl-D-Isoglutamine. Commercial available derivatives of MDP include Muramyldipeptide with a C18 fatty acid chain, MurNAc-Ala-D-isoGln-Lys, naturally released by Lactobacillus salivarus, MurNAc-Ala-D-isoGln-L-Lys(D-Asn), naturally released by *Lactobacillus acidophilus,* and murabutide.

For the reasons already mentioned with respect to the first aspect of the invention, the mammalian stem cells likely to be treated by agonists of NOD2 are preferably human stem cells, either with a view to further re-implanting or re-infusing them into human beings, or with a view to undertaking studies regarding these cells.

With a view to re-implanting or re-infusing stem cells, which have been cultured in presence of an agonist of NOD2, the safety of the agonist to be used should be verified. In this respect, it must be noted that insofar as MDP is a natural compound, present in the cell wall of bacteria, this compound is particularly well suited, as this is a compound naturally present in the mammalian body.

Other stem cells are murine stem cells or rat stem cells, as these mammals can be easily used in studies. Other types of mammal are also envisaged in the frame of the present invention. All these preferred embodiments are detailed with respect to the different other aspects of the invention.

All the preferred embodiments detailed in the section dealing with the therapeutic applications of agonist of NOD2 in cytoprotection of stem cells are also applicable in the context of the different uses mentioned above.

According to a third aspect, the invention is also directed to different products incorporating one or more agonists of NOD2, suitable for the culture, manipulation, preservation or transport of adult vertebrate stem cells, especially adult mammalian stem cells. Such products are indeed particularly appropriate in order to protect stem cells against mortality or loss of multiplication and differentiation capacity, during any manipulation step.

The invention is especially directed to a cell culture medium specifically dedicated to the culture of mammalian adult stem cells, especially to intestinal stem cells, comprising an agonist of NOD2 as cytoprotectant. It may also be a medium suitable for freeze procedure, or for transport procedure, encapsulation, transformation, transduction, long-term preservation, harvest, re-implantation, re-infusion or for any other manipulation step of these cells.

The invention also concerns a medium specifically designed for stem cells for retaining the undifferentiated state of these cells, *inter alia* a medium devoid of differentiation factors. Indeed, during transplantation procedure of stem cells, it is generally imperative to retain the multipotency of the harvested stem cells until reimplantation. In this case, an appropriate medium or culture medium is for undifferentiated cells and does not contain factor inducing differentiation of stem cells; such a medium, devoid of factor inducing differentiation of stem cells; advantageously comprises an agonist of NOD2 according to the invention.

The medium can be liquid, semi-solid or solid. Nutrients and other compounds may also be part of such a medium. Antibiotics can also be added, at suitable concentrations.

The concentration of agonist of NOD2 in the medium, especially in a culture medium, is preferably between 1ng/ml and 100 µg/ml, preferably between 10 ng/ml and 20 ug/ml.

Such a medium or culture medium can be advantageously added to a matrigel.

The present invention also concerns a cell support or a cell matrix or matrigel, suitable for mammalian adult stem cells culture, said support comprising an agonist of NOD2 as cytoprotectant. According to another embodiment, this cell support or a cell matrix or matrigel does not comprise factors inducing differentiation of stem cells.

In the different embodiments described above regarding cell culture medium, cell support, matrix or matrigel, the NOD2 agonist is as described with respect to the other aspects of the invention. NOD2 agonist is therefore preferably the muramyl-dipeptide (N-acetylmuramyl-L-Alanyl-D-Isoglutamine), a derivative thereof, especially derivatives of MDP of formula (Ia), *inter alia* N-Glycolyl- Muramyl dipeptide. Example of such a derivative is for example 6-O-acyl derivatives of Muramyl dipeptide, especially 6-O-stearoyl-N-Acetyl- muramyl-L-Alanyl-D-Isoglutamine according to formula (III) below. Other commercially available derivatives include, Muramyldipeptide with a C18 fatty acid chain, MurNAc-Ala-D-isoGln-Lys (naturally released by Lactobacillus salivarus), MurNAc-Ala-D-isoGln-L-Lys(D-Asn) (naturally released by *Lactobacillus acidophilus)* and murabutide. Other derivatives can be envisaged by the skilled person in the art without specific difficulties.

According to a fourth aspect, the present invention is directed to different processes, taking advantage of the cytoprotection provided to adult stem cells by agonists of NOD2.

The present invention is *inter alia* directed to an *in vitro* screening process for identifying molecules capable of increasing the autonomous capacity of survival, multiplication and differentiation of mammalian adult stem cells, especially in response to a stress. This process preferably comprises a step of providing cells expressing NOD2 protein, either naturally or after activation or transfection of a transgene under an appropriate promoter. Preferably, the expressing cells are from the same organism as the stem cells for which a cytoprotectant is screened. The process comprises also a step of adding a test molecule to said NOD2 expressing cell; this addition step may be addition to the culture medium, or introduction directly into the cells.

The process also comprises a step of detection of an interaction between NOD2, expressed in the cells, and the test molecule.

This interaction can be detected by any appropriate means for measuring the activation of NOD2 in the cell. Depending on the cell type, the activation of NOD2 can be detected for example by IL-8 release detection, or by NF-κB pathway activation.

Any other pathways, known to be specific to NOD2 activation, can be used for detecting the activation of NOD2 by a test molecule. By this process, a test molecule can be identified, which can then be used as cytoprotectant for stem cells.

The test molecule can be any kind of molecule, either naturally occurring or chemically synthesized. Preferably, a test molecule is a derivative of Muramyl-dipeptide (N-acetylmuramyl-L-Alanyl-D-Isoglutamine), especially a molecule according to formula (Ia), or a molecule capable of releasing MDP in intracellular conditions, or capable of releasing a functional equivalent of MDP.

A test molecule may also consist in the fusion of two molecules, for example MDP with a further molecule. Said further molecule may indeed either ease the transport inside stem cells, or may induce transduction, or may help targeting specifically the stem cells, or may add any function which could be beneficial.

In a further embodiment, the present invention is directed to an *in vitro* screening method for identifying molecules capable increasing the autonomous capacity of survival, without loss of multiplication and differentiation capacities of mammalian adult stem cells, especially in response to a stress.

According to this embodiment, a test molecule is added *in vitro* to adult mammalian stem cells and the survival rate and differentiation potential of the stem cells after addition of the test molecule is monitored or assayed, in the following hours or days. The survival rate and differentiation potential is advantageously observed in condition allowing multiplication and differentiation of the stem cells under optimal conditions. Alternatively, the survival rate and maintaining of the differentiation capacity are observed in response to a stress, for example a chemical stress, an oxidative stress, a pharmacological stress, a physical or physiopathological stress, irradiation, infection,...

According to a preferred embodiment of this screening process, the adult stem cells are intestinal stem cells. For this cell type, the survival rate and differentiation potential is preferably assayed by determining the number of organoids formed in the following days, for example after one, two, three, four, five days or more. Examples of implementation of this process are detailed in the experimental section and the specific components to be added to the culture medium to favor organoids formation, are detailed accordingly. The stem cells can advantageously be seeded with Paneth cells, in order to increase organoids formation.

The molecules to be tested according to this *in vitro* process are the same as those detailed with regard to the preceding *in vitro* process.

These screening processes may advantageously be carried out for identifying derivatives of MDP, for example derivatives according to formula (Ia), with improved functionality, or fewer side effects, or simpler or less expensive process of synthesis, or any other improved property. Alternatively, these screening processes may also be carried out in order to screen a small molecule bank, with chemical structures totally unrelated to MDP, in order to identify novel classes of MDP agonists.

### Legend of Figures:

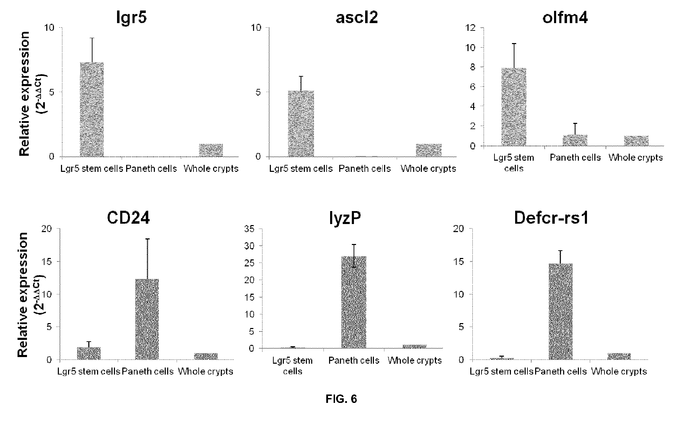

**FIG.1****: Growth curve of organoids.**
   FIG 1A: Fig. 1A is the picture of an organoid, grown from intestinal crypt. This is a structure delineated by a definite monolayer of cells.
   FIG. 1B: Fig. 1B represents a typical growth curve of the number of organoids upon stimulation by MDP, different other MAMPs, and without stimulation (control). The curves depicted are the results from one experiment, but are representative of the typical growth curve.
**FIG .2****: Effects of MAMPs on organoids formation.**
   Fig 2 illustrates the fold change in number of organoids, formed from extracted crypts, depending on the MAMPs applied for stimulation. The average number of non-stimulated organoids (control) is around 50, and the fold change value of the control is fixed to 1. The fold change upon the various MAMPs applied is thus by reference to non-stimulated organoids.
   The results presented in Fig. 2 are the average of 5 different independent experiments. The fold change is measured at day 4.
   As can be seen from this figure, PGN (peptidoglycan) and MDP induce the formation of higher amount of organoids.
   The size of the organoids is the same as the size of the non-stimulated ones.
**FIG .3****: proliferation assays.**

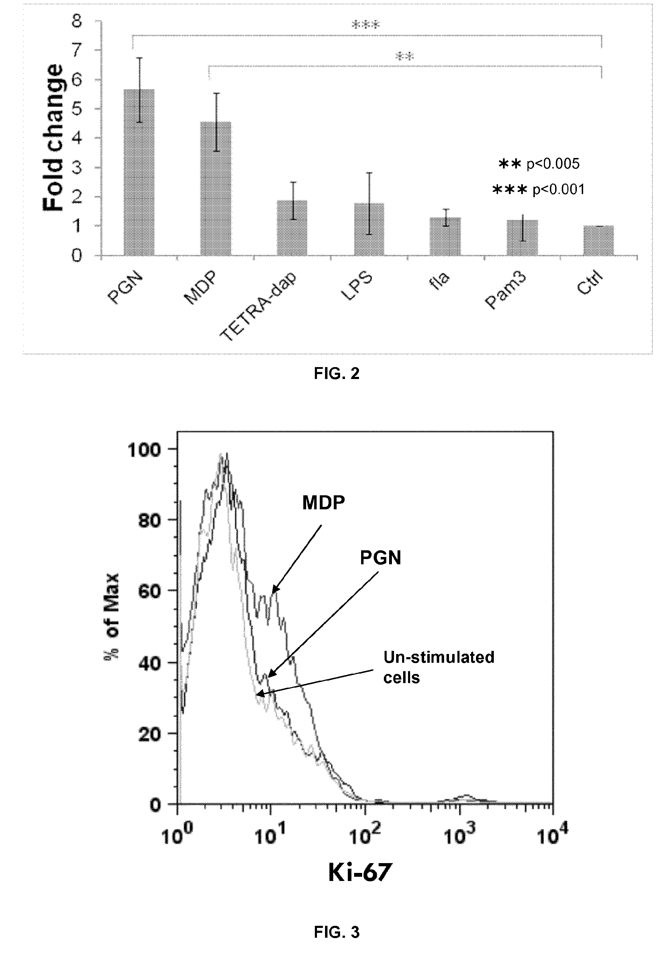

   Fig.3 illustrates the rate of Ki-67 expression upon different stimulation conditions, Ki-67 being a marker for proliferative cells. No differences in the rate of proliferation between treated and un-treated organoids can be seen.
**FIG .4****: Organoids from Nod1 and Nod2 KO mice.**

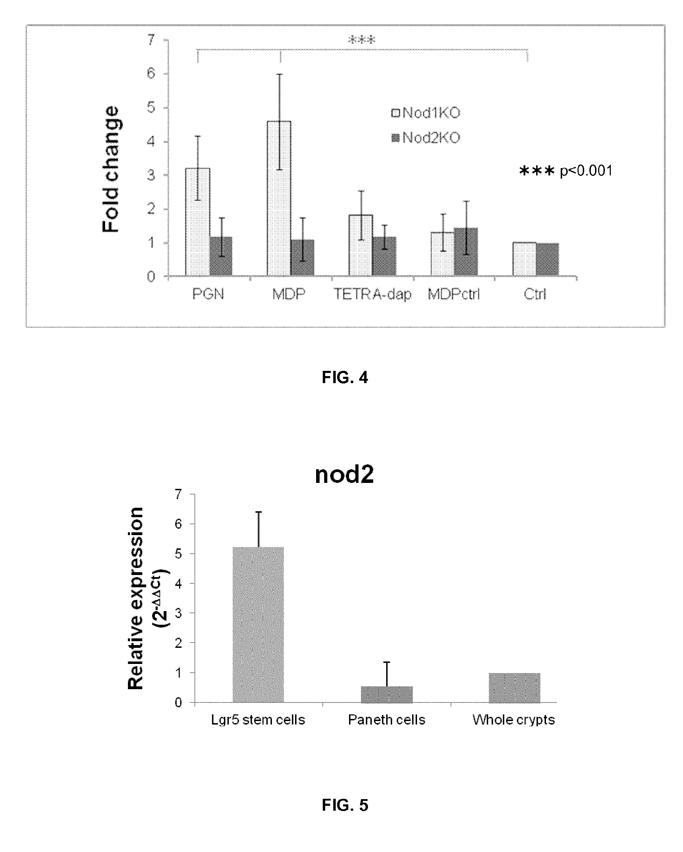

   Fig. 4 illustrates the fold change in number of organoids, formed from extracted crypts, from mice knocked out for *Nod1* or *Nod2* genes, upon different MAMPs stimulation conditions. By definition, the fold change of the untreated organoids is set to 1, and the fold changes upon the various stimulation conditions applied are by reference to the non-stimulated organoids.
**FIG .5****: Nod2 gene expression**
   Fig. 5 illustrates the Nod2 gene expression in Lgr5-expressing stem cells, in Paneth cells and in the whole crypts. The level of expression of Nod2 gene in whole crypts is arbitrarily fixed to "1".
   As can be inferred from the results of figure 5, the level of nod2 mRNA is 5 times more in stem cells than in Paneth cells.
**FIG .6****: Markers of stem cells and of Paneth cells.**
   The relative level of expression of different markers is illustrated in figure 6. As for figure 5, the level of expression for the different markers is arbitrarily fixed to "1" at the level of the whole crypts.
   As can be seen from this figure, Igr5, ascl2 and olfm4 which are specific markers of intestinal stem cells are preferentially expressed in intestinal stem cells extracted from crypts, whereas CD24, lyzP and Defcr-rs1 are mainly expressed in Paneth cells, thus confirming the nature of these cells.
**FIG .7****: Markers of stem cells and of Paneth cells in WT and Nod2 KO cells.**

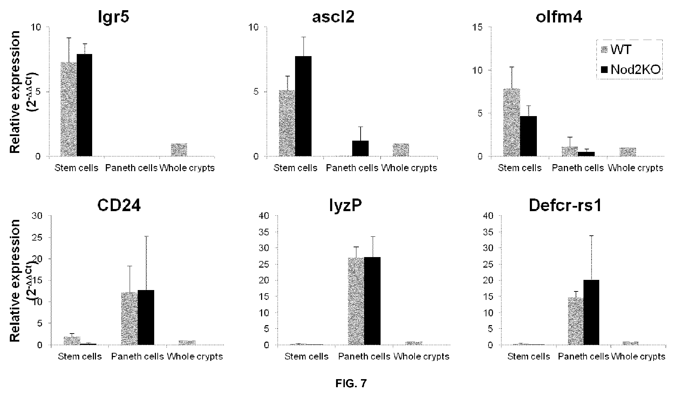

   Fig. 7 illustrates results similar to those presented in fig .6, for crypts extracted from WT mice and from mice KO for Nod2 gene.
**FIG .8****: Single cells stimulation.**

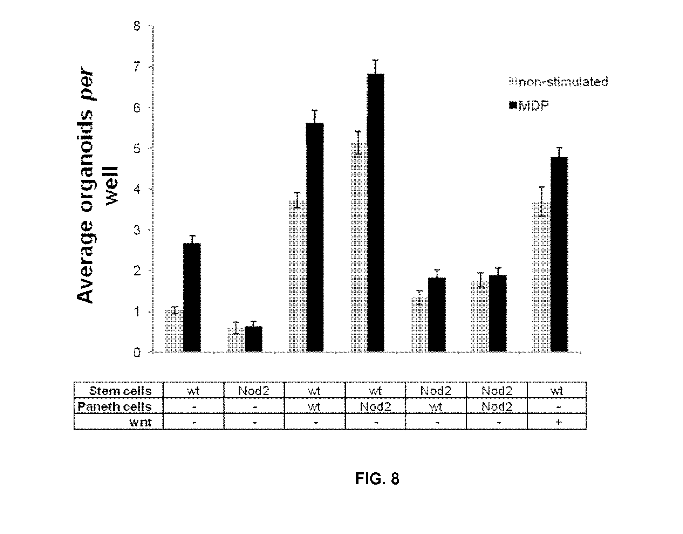

   Fig. 8 depicts the average number of organoids formed per well, depending on the cell types seeded in the well, without stimulation, or under stimulation with MDP. 500 cells were seeded per well in a 96-wells-plate.
   Stem cells are either wild type or KO for the nod2 gene; they are seeded in all wells.
   Paneth cells are also either wild type or KO for the nod2 gene; They are present in the 3^{rd} to 6^{th} series of experiments and absent in the 1^{st}, 2^{nd} and 7^{th} series of experiments.
   In the last (7^{th}) series of experiments, Wnt is added.
**FIG .9****: Inducing stress to stem cells.**

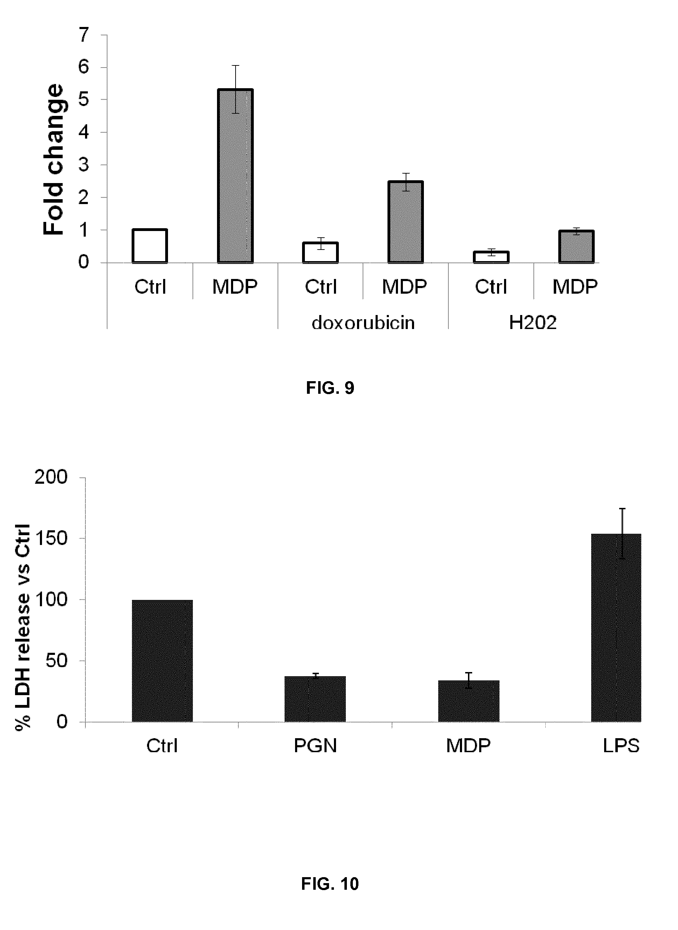

   Fig.9 illustrates the fold change in number of organoids, formed from extracted crypts, upon stimulation with MDP or un-treated, with or without application of a specific stress, either Doxorubicin or H₂O₂. By definition, the fold change of the untreated organoids is set to 1 in the absence of any additional stress, and the fold changes upon the various stimulation or stress conditions applied are by reference to the non-stimulated non-stressed organoids.
   Doxorubicin (1 µM) or H₂O₂ (200µM) are added during the embedding of the crypts in the matrigel. Doxorubicin (DOX) belongs to the anthracycline class of chemotherapeutic agents and works by intercalating DNA. Doxorubicin has been shown to preferentially attack the cells at the bottom of the crypt.
   As can be seen from Fig. 9, MDP is able to protect stem cells.
**FIG .10****: LDH release.**
   Fig. 10 illustrates the percentage of LDH release from crypts treated by different MAMPs, by reference to untreated crypts (corresponding to the control, value set to 100).
   As can be deduced from the results of Fig. 10, the stimulation with PGN or MDP results in a higher viability of the crypts, by reference to untreated crypts.
**FIG .11****: Caspase-3 activation.**

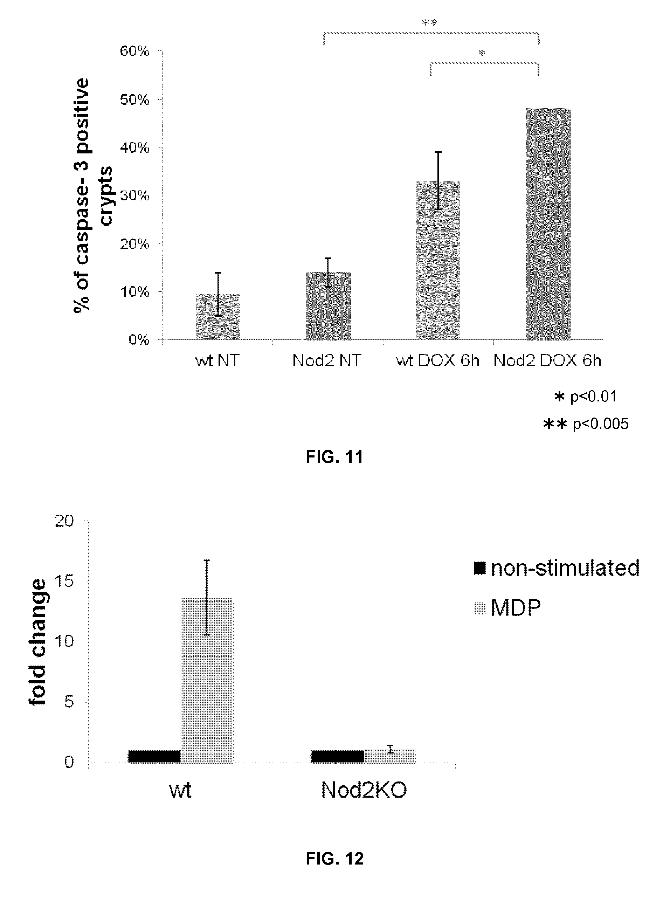

   Fig.11 depicts the percentage of caspase-3 positive crypts in different conditions. The results are obtained after 6 hours.
   Line 1: WT, non-treated mice. Line 2: Nod2 KO mice, non-treated. Line 3: WT mice, treated with doxorubicin during 6 hours. Line 4: Nod2 KO mice, treated with doxorubicin during 6 hours.
   It can be observed that the absence of Nod2 induced more death in the crypts.
**FIG .12****: Organoids from Doxorubicin-treated mice.**
   Fig. 12 illustrates the fold change in number of organoids, formed from extracted crypts from either WT or Nod2 KO mice, 72 hours after treatment with doxorubin, upon stimulation with MDP or un-treated.

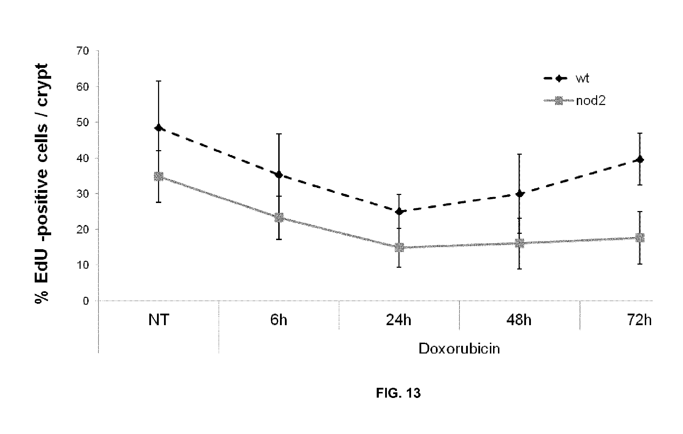

**FIG. 13****: Proliferation index.**
   Fig. 13 illustrates the percentage of EdU-positive cells per crypt, in wild-type or Nod2 KO mice, after 2 hours of EdU (5-ethynyl-2-deoxyuridine) treatment.
   In WT mice, the crypts are prone to regenerate.

By definition, the fold change of the untreated organoids, extracted from WT mice, is set to 1, and the fold changes upon the various stimulation conditions are by reference to the non-stimulated WT organoids.

As can be seen from this figure, upon stress (namely doxorubicin), the stem cells are actively reactivated by the MDP.

### Experimental Section

### EXAMPLE 1: Material and methods

### Mice

B6.129P2-*Lgr5*^{*tm1(cre*/*ESR1)Cle*}/J mice (Lgr5-EGFP) (Barker et al., 2007) were purchased from The Jackson Laboratories. Card4/Nod1 (Nod1 KO) mice were generated by Millenium pharmaceuticals Boston. Card15/Nod2 deficient C57BL/6J (Nod2 KO) (Barreau et al., 2007) were provided by J.-P. Hugot (Hôpital Robert Debré, Paris, France). All mice were kept in specific pathogen-free conditions. When indicated the mice were injected intraperitoneally with 200 mg of EdU (Invitrogen) 2hrs prior to sacrifice or with 10 mg/kg doxorubicin hydrochloride (Sigma) and sacrificed at indicated time points.

### Crypts isolation and organoids formation

Intestinal crypts were obtained following a protocol already described (Sato et al., 2009). After isolation small intestines were isolated, flushed with cold PBS followed by 0.3% bleach and again PBS, opened longitudinally and the villi were removed. The tissues were cutted in small pieces, washed in 5 mM PBS/EDTA for 5 min, and subsequently incubated in fresh 5 mM PBS/EDTA for 30 min on ice. After severals vigourous shakings the crypts, released in the supernatant, were passed through a cell strainer 70 µm (BD Falcon), spun down at 800 rpm, resuspended in DMEM and counted. The volume corresponding to 500 crypts was distributed in two tubes per condition, and spun down again. The pellet of crypts was or not incubated with the following microbe-associated molecular patterns (MAMPs) for 10 min at room temperature: 10 µg/ml Soluble sonicated peptidoglycan from *E*. *coli* K12 (PGN), 10 µg/ml Muramyl-dipeptide (MDP), 10 µg/ml MDP rhodamine, 10 µg/ml MDP control (MDP-ctrl), 1 µg/ml Lipopolysaccharide (LPS), 500 ng/ml Lipoprotein (Pam3), 10 ng/ml Flagellin (Fla) (all purchased from InvivoGen), 10 µg/ml MurNAc-Tetra(DAP) (Tetra-dap) (kindly provided by Dominique Mengin-Lecreulx). The crypts were embedded in 50 µl of growth factor reduced matrigel (BD Biosciences) in a 24-well plates, incubate at 37°C 20 min and overload with 500 µl of crypts medium (CM) as described previously (Sato et al 2009). Briefly, Advanced DMEM/F12 was supplemented with 100 U/ml penicillin/streptomycin, 10 mM Hepes, 1x N2, 1x B27 (all from Gibco), 50 ng/ml EGF (Peprotech), 100 ng/ml Noggin (Peprotech), 500 ng/ml R-spondin1 (R&D). The medium was exchanged every 4 days. To induce toxic stress to the organoids, 1 µM Doxorubicin hydrochloride (from Sigma) or 200 µM H₂O₂ were added to the medium upon the embedding of the crypts into the matrigel.

The number of organoids was evaluating with an inverted microscope equipped with thermostatic chamber (temperature and CO₂).

### Sorting and culture of single cells

Isolated crypts from Lgr5-EGFP mice or Nod2KO mice were incubated in HBSS w/o Ca⁺² and Mg²⁺ supplemented with 0.3 U/ml Dispase (BD Biosciences), 0.8 U/ml DNase (Sigma) and 10 µM Y-27632 (Sigma) for 30 min at 37°C.

Dissociated cells were washed with 1% PBS/BSA and stained with CD24-APC antibody (clone M1/69 BioLegend) and EpCam Pe-Cy7 (clone G8.8 BioLegend) for 20 min at 4°C, resuspended in crypts medium supplemented with 1 µM N-acetyl-L-cystein, 10 µM Jag-1 (Anaspec) and 10 µM Y-27632 (single cells medium-SCM) and filtered with a 20 µm mesh and analyzed with MoFlo legacy (Beckman Coulter).

Singlets of viable epithelial cells were gated by negative staining for propidium iodide and positive staining for EpCam. The stem cells and Paneth cells were isolated respectively as GFP ^{hi+} population, and GFP⁻CD24^{hi+}SSC^{hi} population.

Sorted cells were collected or in SCM for culturing experiments or in RNAprotect Cell Reagent (Qiagen) for the RNA extraction.

Stem cells and Paneth cells derived from Lgr5-EGFP or Nod2KO mice were cultured alone or in different combinations. 500 cells from each condition were re-suspended in 100 µl of single cells medium with or without MAMPs and, when indicated, with wnt3 (R&D), seeded in 96-wells round-bottom plates and incubated on ice for 15 min. The plate was spun at 300g for 5 min and 10 µl of matrigel was added in each well. The number of organoids was counting after 1 week.

### Real time PCR

Total RNA was extracted with the RNeasy Micro Kit (Qiagen) and the cDNA was made with *SuperScript* II Reverse Transcriptase (Invitrogen) and oligo(dT)12-18 primer (Invitrogen) as recommended by the suppliers. The following primers (purchased from Invitrogen) were used:
- Ascl2,: 5'- AAGCACACCTTGACTGGTACG- 3' (SEQ ID N°3) and 5'-AAGTGGACGTTTGCACCTTCA-3' (SEQ ID N°4);
- b2m,: 5'-TCAGTCGTCAGCATGGCTCGC-3' (SEQ ID N°5) and 5'-CTCCGGTGGGTGGCGTGAGTAT-3' (SEQ ID N°6);
- CD24,: 5'-CGAGCTTAGCAGATCTCCACT-3' (SEQ ID N°7) and 5'-GGATTTGGGGAAGCAGAAAT-3' (SEQ ID N°8);
- defcr-rs1,: 5'-AAGAGACTAAAACTGAGGAGCAGC- 3' (SEQ ID N°9) and 5'-CGACAGCAGAGCGTGTA-3' (SEQ ID N°10);
- Lgr5,: 5'-GACAATGCTCTCACAGAC-3' (SEQ ID N°11) and 5'-GGAGTGGATTCTATTATTATGG-3' (SEQ ID N°12);
- LyzP,: 5'-GAGACCGAAGCACCGACTATG- 3' (SEQ ID N°13) and 5'-CGGTTTTGACATTGTGTTCGC-3' (SEQ ID N°14);
- nod2,: 5'-GGAGTGGAACAGCTGCGACCG-3' (SEQ ID N°15) and 5'-GCACACTCAACCAGCGTGCG-3' (SEQ ID N°16);
- Olfm4,: 5'-TGGGCAGAAGGTGGGACTGTGT- 3' (SEQ ID N°17) and 5'-CGGGAAAGGCGGTATCCGGC- 3' (SEQ ID N°18).

Reactions were run on ABI 7900HT (Applied Biosystems) using Power SYBR Green mix (Applied Biosystems) according with the manufactures instructions. B2M RNA was used as an internal control, and ΔΔCT (cycle threshold) values were calculated.

### Immunohistochemistry

The tissue was processed as already described (Escobar et al., 2011). The small intestines were flushed with PBS and everted on 3-mm OD wooden skewers before fixation in 4% paraformaldehyde (Electron Microscopy Sciences) for 2 h. Intestines were released from the skewers by a longitudinal incision and rolled up using wooden toothpicks, incubated for 2 h in 15% sucrose and overnight at 4°C in 30% sucrose. Rolls were then embedded in OCT compound (VWR) frozen in isopentane, cooled with dry ice, and stored at -80°C. Frozen blocks were cut with a thickness of 7 µm using a CM 3050S cryostat (Leica), and sections were collected on Superfrost plus slides (VWR) and stored at -80°C. Alternatively after the fixation the tissues were embedded in paraffin using standard procedures.

Paneth cells were stained using a rabbit anti-Lysozyme (1:500, Thermo) and Alexa fluor 568 as secondary antibody. DNA was stained by DAPI (1 µg/ml, Molecular Probes). The sections were mounted with Prolong gold antifade (Invitrogen). Images were acquired with confocal microscopy (Leica, SP5).

For the caspase-3 staining, tissue sections were deparaffinized in xylene and then rehydrated in a series of alcohols and PBS. Endogenous peroxidase activity was removed by incubating the sections in 3% hydrogen peroxide for 10 min. Antigen retrieval was done by boiling the sections in 10 mM sodium citrate buffer for 20 min. Sections were permeabilized for 10 min with 0.5% triton X-100, blocked with ultra V block (Labvision) 5 min and incubated overnight with a rabbit cleaved Caspase-3 antibody (Cell Signaling Technology, 1:200). Dako EnVision-system HRP anti-rabbit (Dako) 40 min was then applied and the reaction developed with DAB (Dako). Slides were counterstained with Mayer hematoxylin and mounted with Aquatex (Merck). The sections were imaging with Mirax scanner (Zeiss).

### Cytotoxicity assay

Cultures of organoids were analyzed for the release of lactate dehydrogenase (LDH) using Promega CytoTox 96 kit according to provider's instructions. The absorbance was mesaured with the Tecan Sunrise microplate reader and the ratio between the LDH released into the supernatant and the total LDH in cell lysate was measured.

### Proliferation assay

The supernatant was removed and the organoids were dissociated using dispase (BD Bioscience). The cells were fixed with 2% PFA, permeabilized with 0.1% triton X-100 and stained with 1:100 dilution of rabbit polyclonal anti Ki-67 antibody (Abcam) for 1h. Alexa Fluor 647 was used as a secondary antibody. Data were recorded using FACSCalibur flow cytometer (BD) and analyzed with Flowjo software.

### EXAMPLE 2: Results

### Summary:

The present inventors have discovered a NOD2-dependant pathway of cytoprotection of intestinal stem cells, in the presence of muramyl-dipeptide, the best known NOD2-agonist. This is a totally novel function of the NOD signaling pathway that illuminates the pathogenesis of IBDs (inflammatory bowel diseases) like Crohn disease, and offers a novel approach to maintain stem cells alive in conditions of manipulation.

The intestinal mucosa surface is continuously exposed to a complex and dynamic community of microorganisms, the microbiota. These microbes establish symbiotic relationships with their hosts, and this interaction plays a crucial role in the maintenance of intestinal epithelial homeostasis. The bacteria present in the lumen release microbial components, namely MAMPs for microbe-associated molecular patterns, which access the intestinal crypts, a sensitive region responsible for epithelial regeneration where intestinal stem cells are located. Using a newly developed *in vitro* method described by Clevers group (Sato et al., 2009), the inventors cultured intestinal crypts organoids testing several MAMPs. Starting from the first day of culture, they found a higher number of organoids upon stimulation with bacterial peptidoglycan (PGN), especially with MDP, a PGN subunit present in Gram positive and Gram negative bacteria. On the contrary the inventors did not observe any differences with other PGN motifs such as Tetra-dap. This result is due to the fact that Tetradap and MDP are respectively recognized by two different murine receptors, Nod1 and Nod2 (Magalhaes et al., 2005; Girardin et al., 2003). Only Nod2 seems to be involved in the observed phenotype. Indeed comparing the effect of PGN or MDP on crypts extracted from Nod1 or Nod2 deficient mice, the inventors observed the same effect seen in the wt crypts in the Nod1 KO mice. As expected, organoids produced from Nod2 KO mice were not affected by the presence of

### MDP.

Using Lgr5-EGFP-ires-creERT2 mice, the inventors were able to sort intestinal stem cell (SC) and cultivate them to produce organoids. When the Paneth cells were added to SC the rate of organoids formation increased. Adding MDP to the medium of single SC or into a co-culture of SC and Paneth cells the inventors always observed an increase in numbers of organoids. It is known from the literature that Nod2 is expressed at the bottom of the intestinal crypts, at the level of Paneth cells. However, the higher survival of organoids was not associated to the expression of Nod2 in Paneth cells. In fact co-culturing Paneth cells isolated from Nod2 KO mice with wt stem cells, the increase in the number of organoids due to the presence of MDP was still present.

Analyzing the gene expression in those cells, the inventors found that not only the Paneth cells express the nod2 gene but also the Igr5 positive stem cells. Moreover nod2 gene is expressed 5 times more in SC compared to Paneth cells. This explains the direct effect of MDP on stem cells.

Organoids produced upon stimulation with PGN had a size similar to the non-stimulated ones, meaning that the rate of proliferation is the same. Therefore MDP has a cyto-protective effect on isolated intestinal crypts. In fact, crypts stimulated with MDP release less LDH compared to untreated organoids.

MDP shed by the luminal microbiota can thus interact with the Nod2 receptor expressed by Paneth cell and stem cells. The Paneth cells could be induced to release factors like EGF, TGF-a, Wnt3 required to support the stem cells. On the other hand MDP interacting directly with stem cells increases their survival.

### Detailed Results:

Recently a culture system for intestinal crypts has been described (Sato et al., 2009). Embedding isolated crypts in matrigel and adding growth factors, leads to three-dimensional structures, called organoids. Organoids recapitulate the crypt-villus architecture, with an internal lumen, the different epithelial lineages and stem cells located in surface protrusions, corresponding to novel crypts.

**Organoids stimulation**. To evaluate the effect of bacterial products on intestinal crypts the inventors grew organoids in the presence of different purified or synthetic microbe-associated molecular patterns (MAMPs). By definition, MAMPs are common to all the bacteria, pathogenic and not pathogenic, and can be released from the microbiota normally present in the gut (Garret et al., 2010). To allow MAMPs to be entrapped in the lumen of organoids they added them prior to embedding of the crypts into the matrigel. To check that the MAMPs localized inside the organoid lumen, they used, for instance, MDP-rhodamine in order to monitor the location of this molecule upon stimulation. They observed that the red signal of the MDP localized inside the organoid lumen.

To monitor if pattern recognition receptors (PRRs), upon recognition of their ligands, affected the occurrence and development of organoids, they assayed different MAMPs. Nod1 and Nod2 receptors were stimulated using soluble sonicated peptidoglycan (PGN); moreover they used Tetra-dap and MDP to discriminate between the two Nod proteins, respectively Nod1 and Nod2. Synthetic lipoprotein (Pam3), lipopolysaccharide (LPS), and flagellin (Fla) were also assayed to respectively stimulate TLR2, TLR4 or TLR5.

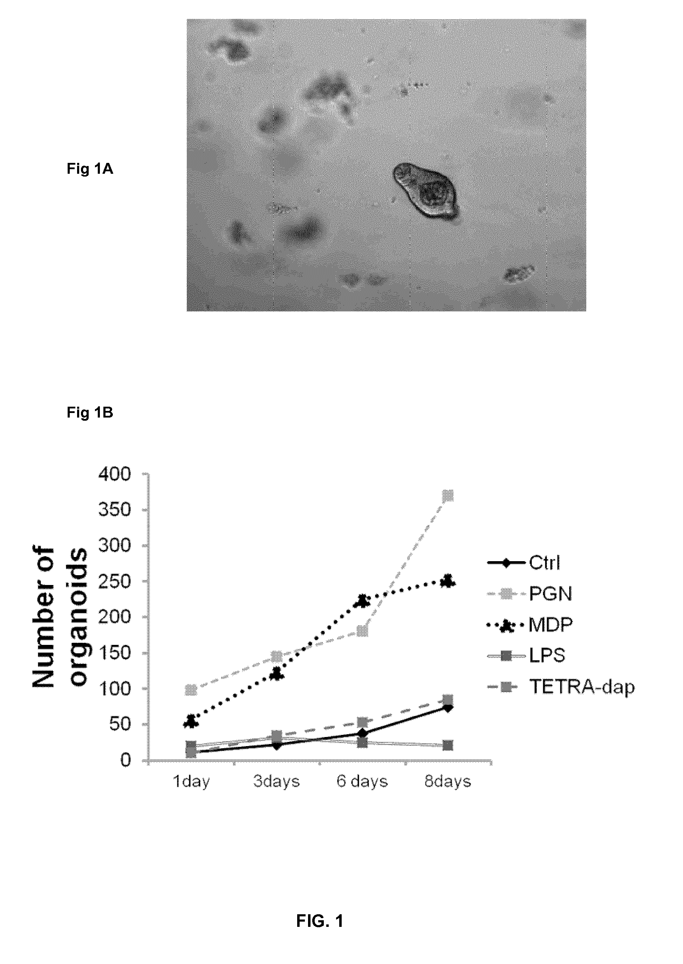

Upon MAMP-stimulation they monitored the number of formed organoids. They considered as organoid a structure delineated by a definite monolayer of cells (**FIG. 1A**). A typical growth curve of the number of organoids upon stimulation is shown in **FIG. 1****B.** Starting from the first day they observed a higher number of organoids in the conditions stimulated with PGN and with MDP, compared to the others, and this difference was maintained, and even increased over the time of the experiments.

They elected to monitor the number of organoids at day 4, the time at which the medium was normally exchanged for long culturing. Upon stimulation with PGN they observed around 5 times more organoids compared with untreated cultures (**FIG. 2**). To distinguish between the role of Nod1 and Nod2, they compared the effect of their dedicated agonists and found the same effect seen with PGN using MDP, the Nod2 agonist, but not with Tetra-dap, the Nod1 agonist.

No differences were observed in the size of the organoids, indicating that stimulation with MAMPs did not affect their rate of proliferation. To confirm this observation, after 4 days of culture, treated or not treated organoids were tested for the expression of Ki-67, a marker for proliferative cells. As shown in **FIG.3** they did not observe any variation in the rate of proliferation between organoids stimulated with PGN or MDP, compared with untreated controls.

To further investigate the effect of PGN, they generated organoids from Nod1 KO and Nod2 KO mice. After stimulation with PGN and MDP, the number of organoids from Nod1 KO mice, was 3 to 5 times higher, compared with untreated ones (**FIG. 4**). To confirm that the effect on the numbers of organoids was MDP-dependent, the inactive form of MDP, the D-D isomer (MDP-ctrl) was tested. No difference was observed with the control samples. They applied the same stimulus to organoids from Nod2 KO mice and did not observe any difference.

It has been shown that in the gut the Nod2 receptor is present at the level of the crypts in mice (Kobayashi et al., 2005). In human intestinal tissues it was demonstrated that Nod2 is expressed in Paneth cells (Ogura et al., 2003; Lala et al., 2003). Moreover an important role was shown for Paneth cells as main constituent of the niche of Lgr5 stem cells in intestinal crypts (Sato et al., 2010). To further investigate the expression of the *nod2* gene in intestinal crypts, stem cell and Paneth cells were sorted, starting from Lgr5-EGFP knock-in mice. Epithelial cells were selected using an anti-Epcam antibody. Stem cells were sorted as GFP "highly positive", CD24 "medium" positive cells, while Paneth cells were isolated as high-SSC and high-CD24 population.

**Purity after sorting**. To confirm the purity of the two cell populations, they performed real time-PCR checking for expression of the respective markers of stem cells and Paneth cells. B2M was used as an internal control gene and ΔΔCt values were calculated to obtain relative expression compared to whole crypt expression. For the stem cells they found high expression of Lgr5 (Barker et al., 2007), Ascl2 (Van der Flier et al, 2009a) and Olfm4 (Van der Flier et al, 2009b), and low expression of CD24. Paneth cells highly expressed CD24 (Sato et al 2009), LyzP and defcr-rs1 (Garcia et al., 2009) (**FIG 6**). Then they analyzed stem cells and Paneth cells for expression of the *nod2* gene. Paneth cells expressed Nod2, as previously reported, but in a lower level compared to stem cells, that showed to have 5 times more Nod2 gene than Paneth cells (see **FIG. 5**).

**Sorting from Nod2 KO mice.** Nod2 KO mice were generated by the disruption of the *Nod2* gene with a EGFP cassette (Barreau et al., 2007). Cells that normally express Nod2 thus became fluorescent. They decided to evaluate the GFP signal at the level of intestinal crypts and verified that the signal was generated by the stem cells and not by the Paneth cells. They stained the Paneth cells with an anti-lysozyme antibody, and observed that the green cells, were interspersed between Paneth cells, exactly in the position of the Lgr5 stem cells. Starting from this observation they decided to extract crypts from Nod2 KO mice and to sort the GFP positive cells to verify that those cells correspond to Lgr5-expressing stem cells. Then they analyzed by RT-PCR the gene expression pattern of the sorted cells, checking the respective markers of stem cells and Paneth cells. They confirmed that GFP positive cells were expressing the markers of stem cells at the level observed in Lgr5-EGFP mice, and the same for the Paneth cells markers (**FIG. 7**).

**Single cells stimulation**. Using the same sorting strategy used to isolate cells for gene analysis, they recovered stem cells and Paneth cells from Lgr5-EGFP and Nod2 KO mice. When GFP positive cells were sorted from Nod2 KO mice and cultivated in appropriate medium, they observed the formation of organoids, meaning that the cells had properties of stem cells (**FIG. 8**). In this condition the average number of organoids was about 1.5 fold lower compared with wt stem cells. No differences were found when MDP was added to stem cells originating from Nod2 KO mice. On the contrary, upon MDP stimulation of wt stem cells, 2.5 more organoids were observed, compared to the unstimulated condition (p<0.0001). Co-culturing Paneth cells, derived from both wt and Nod2 KO mice, with wt stem cells, or adding the wnt ligand to wt stem cells alone, the average of organoids *per* well was around 3, a value similar to the one obtained in MDP-treated wt stem cells. Adding MDP to a co-culture associating wt stem cells and wt Paneth cells they observed again an increase in the average of organoids, around 6 organoids *per* well. The same result was obtained by co-culturing wt stem cells and Nod2 KO Paneth cells, meaning that the increase in the number of organoids upon stimulation was dependent on Nod2 expressed in stem cells. Moreover upon MDP-stimulation, no differences were observed in co-cultures associating Nod2 KO stem cells with wt or Nod2 KO Paneth cells.

**Inducing stress.** To test if MDP had a protective effect on stem cells present in the crypt, they decided to induce a stress targeted to intestinal stem cells. As a drug they used 1 µM doxorubicin hydrochloride (dox), known to be toxic for the stem cell (Dekaney et al., 2009), or 200 µM H₂O₂ to induce an oxidative stress.

After 4 days of treatment they counted 50% less organoids. When the MDP was added to the crypts in presence of doxorubicin, they were able to restore the normal growth of the organoids maintaining the same ratio between stimulated and non-stimulated organoids of 5 times. In H₂O₂ treated samples, the increase in the number of organoids upon MDP-stimulation was still present but in a lower ratio (**FIG.9**).

**Cytoprotection.** Finally to assess if the differences in the number of organoids upon stimulation was dependent from a better survival of the crypts in the presence of MDP, they tested organoid culture for the release of lactate dehydrogenase (LDH). After 4 days of culture in presence of PGN or MDP the organoids produced 50% less LDH to non-stimulated crypts (**FIG. 10**).

**Doxorubicin *in vivo.*** To evaluate if the cytoprotective effect induced by MDP in a Nod2-dependent pathway on stem cells, that they observed *in vitro,* was also observed *in vivo,* they treated wt and Nod2 KO mice with doxorubicin. Mice were injected intraperitoneally with 20 mg/kg doxorubicin. Due to the fluorescent properties of doxorubicin, they could observe in tissue sections that red signal of doxorubicin was colocalizing with the GFP signal of the stem cells (in a Lgr5-EGFP mouse). The percentage of crypts presenting at least one caspase3-positve cell was counted. As shown in **FIG. 11** upon treatment with doxorubicin wt mice presented 30% of the crypts with apoptotic cells, compare to 10% in the non-treated. In the Nod2 KO mice the effect of doxorubicin caused an increase in the percentage of apoptotic crypts, going from 14% in the non-treated to 54% in the doxorubicin injected mice. To evaluate if upon doxorubicin treatment the proliferation index was affected, the number of EdU positive cells per crypts was counted and the percentage versus the total number of cells in the crypts was calculated. Both wt and Nod2 KO mice showed a decrease in the rate of proliferation starting from 6h upon the injection of doxorubicin until 24 h. The Nod2 KO mice showed a low rate of proliferation in the crypts also in the following days, instead at 48h the percentage of proliferative cells in the crypts of wt mice increased again until 72 h reaching almost the same rate observed in the non-treated mice (**FIG. 13**).

After 72 h the intestinal crypts were extracted from the doxorubicin treated mice, and tested for the capacity to form organoids and to respond to MDP. As shown in **FIG. 12** upon MDP stimulation the number of organoids was increasing more than 10 times compare to non-stimulated samples.

### References

Barker, N., van Es, J. H., Kuipers, J., Kujala, P., van den Born, M., Cozijnsen, M., Haegebarth, A., et al. (2007). Identification of stem cells in small intestine and colon by marker gene Lgr5. Nature, 449(7165), 1003-1007.
Barreau, F., Meinzer, U., Chareyre, F., Berrebi, D., Niwa-Kawakita, M., Dussaillant, M., Foligne, B., et al. (2007). CARD15/NOD2 Is Required for Peyer's Patches Homeostasis in Mice. PLoS ONE, 2(6), e523.
Dekaney, C. M., Gulati, A. S., Garrison, A. P., Helmrath, M. A., & Henning, S. J. (2009). Regeneration of intestinal stem/progenitor cells following doxorubicin treatment of mice. AJP: Gastrointestinal and Liver Physiology, 297(3), G461-G470.
Escobar, M., Nicolas, P., Sangar, F., Laurent-Chabalier, S., Clair, P., Joubert, D., Jay, P., et al. (2011). Intestinal epithelial stem cells do not protect their genome by asymmetric chromosome segregation. Nature Communications, 2, 258.
Garcia, M. I., Ghiani, M., Lefort, A., Libert, F., Strollo, S., & Vassart, G. (2009). LGR5 deficiency deregulates Wnt signaling and leads to precocious Paneth cell differentiation in the fetal intestine. Developmental Biology, 331(1), 58-67.
Garrett, W. S., Gordon, J. I., & Glimcher, L. H. (2010). Homeostasis and Inflammation in the Intestine. Cell, 140(6), 859-870.
Girardin, S.E. et at (2001). CARD4/Nod1 mediates NF-kappaB and JNK activation by invasive Shigella flexneri. EMBO Rep.,2:736-742.
Girardin, S. E. (2003). Peptidoglycan Molecular Requirements Allowing Detection by Nod1 and Nod2. Journal of Biological Chemistry, 278(43), 41702-41708.
Kim et al, (2010). Implication of NOD1 and NOD2 for the differentiation of multipotent mesenchymal stem cells derived from human umbilical cord blood. PLoS One. 5(10):e15369 Kobayashi, K. S. (2005). Nod2-Dependent Regulation of Innate and Adaptive Immunity in the Intestinal Tract. Science, 307(5710), 731-734.
Lala, S., Ogura, Y., Osborne, C., Hor, S. Y., Bromfield, A., Davies, S., Ogunbiyi, O., et al. (2003). Crohn's disease and the NOD2 gene: a role for paneth cells. Gastroenterology, 125(1), 47-57.
Macho Fernandez E. et al., (2011). Anti-inflammatory capacity of selected lactobacilli in experimental colitis is driven by NOD2-mediated recognition of a specific peptidoglycan derived muropeptide, Gut, 60: 1050 - 1059
Magalhaes, J. G., Philpott, D. J., Nahori, M.-A., Jéhanno, M., Fritz, J., Bourhis, L. L., Viala, J., et al. (2005). Murine Nod1 but not its human orthologue mediates innate immune detection of tracheal cytotoxin. EMBO reports, 6(12), 1201-1207
Ogura, Y., Lala, S., Xin, W., Smith, E., Dowds, T. A., Chen, F. F., Zimmermann, E., et al. (2003). Expression of NOD2 in Paneth cells: a possible link to Crohn's ileitis. Gut, 52(11), 1591-1597.
Sato, T., Vries, R. G., Snippert, H. J., van de Wetering, M., Barker, N., Stange, D. E., van Es, J. H., et al. (2009). Single Lgr5 stem cells build crypt-villus structures in vitro without a mesenchymal niche. Nature, 459(7244), 262-265.
Stelzner M, Chen DC (2006). To make a new intestinal mucosa. Rejuvenation Res.;9(1):20-5. Tower J (2012). Stress and stem cells. WIREs Dev Biol.doi:10.1002/wdev.56
van der Flier, L. G., van Gijn, M. E., Hatzis, P., Kujala, P., Haegebarth, A., Stange, D. E., Begthel, H., et al. (2009a). Transcription Factor Achaete Scute-Like 2 Controls Intestinal Stem Cell Fate. Cell, 136(5), 903-912.
van der Flier, L. G., Haegebarth, A., Stange, D. E., van de Wetering, M., & Clevers, H. (2009b). OLFM4 Is a Robust Marker for Stem Cells in Human Intestine and Marks a Subset of Colorectal Cancer Cells. YGAST, 137(1), 15-17.

## Claims

1. Agonist of NOD2 for use in therapy for increasing the autonomous capacity of survival of mammalian adult stem cells, without loss of their capacity to multiply and differentiate.

2. The agonist of NOD2 for use according to claim 1, wherein said adult stem cells are intestinal stem cells, mesenchymal stem cells, hematopoietic stem cells or skin stem cells.

3. The agonist of NOD2 for use according to claim 1, wherein the increase of the autonomous capacity of survival, without loss of the capacity to multiply and differentiate, is in response to a stress applied to the stem cells.

4. The agonist of NOD2 for use according to claim 3, wherein said stress is a chemical stress, an oxidative stress, irradiation, a pharmacological stress, a physical or physiopathological stress or an infectious stress.

5. The agonist of NOD2 for use according to claim 3, wherein said stress is induced by chemotherapy, by radiotherapy, by infection or by tissue injury or dissection.

6. The agonist of NOD2 for use according to claim 3, wherein said stress is induced by manipulation of said adult stem cells, during harvest or implantation of these cells.

7. The agonist of NOD2 for use according to claim 6 during transplantation of intestinal stem cells for treating intestinal atrophy, crypt dysfunction or inflammatory bowel diseases, or during transplantation of skin stem cell for accelerating repair of skin and other epithelial wounds, or during bone marrow transplantation.

8. Agonist of NOD2 for use in transplantation of mammalian adult stem cells, for improving engraftment of said cells and tissue regeneration.

9. The agonist of NOD2 for use according to any one of claims 1 to 8, wherein said agonist is Muramyl dipeptide (N-acetylmuramyl-L-Alanyl-D-Isoglutamine) or a chemical derivative thereof, including N-Glycolyl- Muramyl dipeptide, a 6-O-acyl derivative of Muramyl dipeptide, muramyldipeptide with a C18 fatty acid chain, MurNAc-Ala-D-isoGln-Lys, MurNAc-Ala-D-isoGln-L-Lys(D-Asn) and murabutide (N-Acetyl-muramyl-L- Alanyl-D-Glutamin-n-butyl-ester).

10. Use of an agonist of NOD2 for increasing *in vitro* or *ex vivo* the autonomous capacity of survival, without loss of their capacity to multiply and differentiate, of mammalian adult stem cells.

11. Use according to claim 10 , wherein said adult stem cells are intestinal stem cells, mesenchymal stem cells, hematopoietic stem cells or skin stem cells.

12. Use according to claim 10 or 11, in response to a stress applied to the stem cells, wherein said stress is a chemical stress, an oxidative stress, a pharmacological or physical stress, or an infectious stress.

13. Use according to claim 12, wherein said stress is induced by manipulation, culture, sampling or isolation of said adult stem cells.

14. Use according to any one of claims 10 to 13, wherein said agonist is Muramyl-dipeptide (N-acetylmuramyl-L-Alanyl-D-Isoglutamine) or a derivative thereof, including .N-Glycolyl- Muramyl dipeptide, a 6-O-acyl derivative of Muramyl dipeptide, muramyldipeptide with a C18 fatty acid chain, MurNAc-Ala-D-isoGln-Lys, MurNAc-Ala-D-isoGln-L-Lys(D-Asn) and murabutide (N-Acetyl-muramyl-L- Alanyl-D-Glutamin-n-butyl-ester).

15. Culture medium, cell support or matrix for mammalian adult stem cells, comprising an agonist of NOD2 as cytoprotectant, preferably Muramyl-dipeptide (N-acetylmuramyl-L-Alanyl-D-Isoglutamine) or a derivative thereof, including N-Glycolyl- Muramyl dipeptide, a 6-O-acyl derivative of Muramyl dipeptide, muramyldipeptide with a C18 fatty acid chain, MurNAc-Ala-D-isoGln-Lys, MurNAc-Ala-D-isoGln-L-Lys(D-Asn) and murabutide (N-Acetyl-muramyl-L- Alanyl-D-Glutamin-n-butyl-ester).

16. *In vitro* screening process for identifying molecules capable of increasing, in response to a stress, the autonomous capacity of survival of mammalian adult stem cells, without loss of their capacity to multiply and differentiate, comprising:
- Providing intestinal stem cells expressing NOD2 protein;
- Adding a test molecule, and
- Detecting an increase in the number of organoids formed in the presence of the test molecule, under appropriate culture conditions.


SEQUENCE LISTING

<110>  Institut Pasteur

<120>  A NOD2 dependant pathway of cytoprotection of stem cells

<130>  B10046 CA/CS

<160>  18

<170>  PatentIn version 3.3

<210>  1
<211>  1040
<212>  PRT
<213>  Homo sapiens

<400>  1

<210> 2
<211> 1013
<212> PRT
<213> Mus musculus

<400> 2

Met Cys Ser Gln Glu Glu Phe Gln Ala Gln Arg Ser Gln Leu Val Ala
1               5                   10                  15

Leu Leu Ile Ser Gly Ser Leu Glu Gly Phe Glu Ser Ile Leu Asp Trp
            20                  25                  30

Leu Leu Ser Trp Asp Val Leu Ser Arg Glu Asp Tyr Glu Gly Leu Ser
        35                  40                  45

Leu Pro Gly Gln Pro Leu Ser His Ser Ala Arg Arg Leu Leu Asp Thr
    50                  55                  60

Val Trp Asn Lys Gly Val Trp Gly Cys Gln Lys Leu Leu Glu Ala Val
65                  70                  75                  80

Gln Glu Ala Gln Ala Asn Ser His Thr Phe Glu Leu Tyr Gly Ser Trp
                85                  90                  95

Asp Thr His Ser Leu His Pro Thr Arg Asp Leu Gln Ser His Arg Pro
            100                 105                 110

Ala Ile Val Arg Arg Leu Tyr Asn His Val Glu Ala Met Leu Glu Leu
        115                 120                 125

Ala Arg Glu Gly Gly Phe Leu Ser Gln Tyr Glu Cys Glu Glu Ile Arg
    130                 135                 140

Leu Pro Ile Phe Thr Ser Ser Gln Arg Ala Arg Arg Leu Leu Asp Leu
145                 150                 155                 160

Ala Ala Val Lys Ala Asn Gly Leu Ala Ala Phe Leu Leu Gln His Val
                165                 170                 175

Arg Glu Leu Pro Ala Pro Leu Pro Leu Pro Tyr Glu Ala Ala Glu Cys
            180                 185                 190

Gln Lys Phe Ile Ser Lys Leu Arg Thr Met Val Leu Ala Gln Ser Arg
        195                 200                 205

Phe Leu Ser Thr Tyr Asp Gly Ser Glu Asn Leu Cys Leu Glu Asp Ile
    210                 215                 220

Tyr Thr Glu Asn Ile Leu Glu Leu Arg Thr Glu Val Gly Thr Ala Gly
225                 230                 235                 240

Ala Leu Gln Lys Ser Pro Ala Ile Leu Gly Leu Glu Asp Leu Phe Asp
                245                 250                 255

Thr His Gly His Leu Asn Arg Asp Ala Asp Thr Ile Leu Val Val Gly
            260                 265                 270

Glu Ala Gly Ser Gly Lys Ser Thr Leu Leu Gln Arg Leu His Leu Leu
        275                 280                 285

Trp Ala Thr Gly Arg Ser Phe Gln Glu Phe Leu Phe Ile Phe Pro Phe
    290                 295                 300

Ser Cys Arg Gln Leu Gln Cys Val Ala Lys Pro Leu Ser Leu Arg Thr
305                 310                 315                 320

Leu Leu Phe Glu His Cys Cys Trp Pro Asp Val Ala Gln Asp Asp Val
                325                 330                 335

Phe Gln Phe Leu Leu Asp His Pro Asp Arg Val Leu Leu Thr Phe Asp
            340                 345                 350

Gly Leu Asp Glu Phe Lys Phe Arg Phe Thr Asp Arg Glu Arg His Cys
        355                 360                 365

Ser Pro Ile Asp Pro Thr Ser Val Gln Thr Leu Leu Phe Asn Leu Leu
    370                 375                 380

Gln Gly Asn Leu Leu Lys Asn Ala Cys Lys Val Leu Thr Ser Arg Pro
385                 390                 395                 400

Asp Ala Val Ser Ala Leu Leu Arg Lys Phe Val Arg Thr Glu Cys Gln
                405                 410                 415

Leu Lys Gly Phe Ser Glu Glu Gly Ile Gln Leu Tyr Leu Arg Lys His
            420                 425                 430

His Arg Glu Pro Gly Val Ala Asp Arg Leu Ile Gln Leu Ile Gln Ala
        435                 440                 445

Thr Ser Ala Leu His Gly Leu Cys His Leu Pro Val Phe Ser Trp Met
    450                 455                 460

Val Ser Arg Cys His Arg Glu Leu Leu Leu Gln Asn Arg Gly Phe Pro
465                 470                 475                 480

Thr Thr Ser Thr Asp Met Tyr Leu Leu Ile Leu Gln His Phe Leu Leu
                485                 490                 495

His Ala Ser Pro Pro Asp Ser Ser Pro Leu Gly Leu Gly Pro Gly Leu
            500                 505                 510

Leu Gln Ser Arg Leu Ser Thr Leu Leu His Leu Gly His Leu Ala Leu
        515                 520                 525

Arg Gly Leu Ala Met Ser Cys Tyr Val Phe Ser Ala Gln Gln Leu Gln
    530                 535                 540

Ala Ala Gln Val Asp Ser Asp Asp Ile Ser Leu Gly Phe Leu Val Arg
545                 550                 555                 560

Ala Gln Ser Ser Val Pro Gly Ser Lys Ala Pro Leu Glu Phe Leu His
                565                 570                 575

Ile Thr Phe Gln Cys Phe Phe Ala Ala Phe Tyr Leu Ala Val Ser Ala
            580                 585                 590

Asp Thr Ser Val Ala Ser Leu Lys His Leu Phe Ser Cys Gly Arg Leu
        595                 600                 605

Gly Ser Ser Leu Leu Gly Arg Leu Leu Pro Asn Leu Cys Ile Gln Gly
    610                 615                 620

Ser Arg Val Lys Lys Gly Ser Glu Ala Ala Leu Leu Gln Lys Ala Glu
625                 630                 635                 640

Pro His Asn Leu Gln Ile Thr Ala Ala Phe Leu Ala Gly Leu Leu Ser
                645                 650                 655

Gln Gln His Arg Asp Leu Leu Ala Ala Cys Gln Ile Ser Glu Arg Val

660                 665                 670

Leu Leu Gln Arg Gln Ala Arg Ala Arg Ser Cys Leu Ala His Ser Leu
        675                 680                 685

Arg Glu His Phe His Ser Ile Pro Pro Ala Val Pro Gly Glu Thr Lys
    690                 695                 700

Ser Met His Ala Met Pro Gly Phe Ile Trp Leu Ile Arg Ser Leu Tyr
705                 710                 715                 720

Glu Met Gln Glu Glu Gln Leu Ala Gln Glu Ala Val Arg Arg Leu Asp
                725                 730                 735

Ile Gly His Leu Lys Leu Thr Phe Cys Arg Val Gly Pro Ala Glu Cys
            740                 745                 750

Ala Ala Leu Ala Phe Val Leu Gln His Leu Gln Arg Pro Val Ala Leu
        755                 760                 765

Gln Leu Asp Tyr Asn Ser Val Gly Asp Val Gly Val Glu Gln Leu Arg
    770                 775                 780

Pro Cys Leu Gly Val Cys Thr Ala Leu Tyr Leu Arg Asp Asn Asn Ile
785                 790                 795                 800

Ser Asp Arg Gly Ala Arg Thr Leu Val Glu Cys Ala Leu Arg Cys Glu
                805                 810                 815

Gln Leu Gln Lys Leu Ala Leu Phe Asn Asn Lys Leu Thr Asp Ala Cys
            820                 825                 830

Ala Cys Ser Met Ala Lys Leu Leu Ala His Lys Gln Asn Phe Leu Ser
        835                 840                 845

Leu Arg Val Gly Asn Asn His Ile Thr Ala Ala Gly Ala Glu Val Leu
    850                 855                 860

Ala Gln Gly Leu Lys Ser Asn Thr Ser Leu Lys Phe Leu Gly Phe Trp
865                 870                 875                 880

Gly Asn Ser Val Gly Asp Lys Gly Thr Gln Ala Leu Ala Glu Val Val
                885                 890                 895

Ala Asp His Gln Asn Leu Lys Trp Leu Ser Leu Val Gly Asn Asn Ile
            900                 905                 910

Gly Ser Met Gly Ala Gln Ala Leu Ala Leu Met Leu Glu Lys Asn Lys
        915                 920                 925

Ser Leu Glu Glu Leu Cys Leu Glu Glu Asn His Ile Cys Asp Glu Gly
    930                 935                 940

Val Tyr Ser Leu Ala Glu Gly Leu Lys Arg Asn Ser Thr Leu Lys Phe
945                 950                 955                 960

Leu Lys Leu Ser Asn Asn Gly Ile Thr Tyr Arg Gly Ala Glu Ala Leu
                965                 970                 975

Leu Gln Ala Leu Ser Arg Asn Ser Ala Ile Leu Glu Val Trp Leu Arg
            980                 985                 990

Gly Asn Thr Phe Ser Leu Glu Glu  Ile Gln Thr Leu Ser  Ser Arg Asp
        995                 1000                 1005

Ala Arg  Leu Leu Leu
    1010

<210>  3
<211>  21
<212>  DNA
<213>  Mus musculus

<400>  3
aagcacacct tgactggtac g                                                21

<210>  4
<211>  21
<212>  DNA
<213>  Mus musculus

<400>  4
aagtggacgt ttgcaccttc a                                                21

<210>  5
<211>  21
<212>  DNA
<213>  Mus musculus

<400>  5
tcagtcgtca gcatggctcg c                                                21

<210>  6
<211>  22
<212>  DNA
<213>  Mus musculus

<400>  6
ctccggtggg tggcgtgagt at                                               22

<210>  7
<211>  21
<212>  DNA

<213>  Mus musculus

<400>  7
cgagcttagc agatctccac t                                                21


<210>  8
<211>  20
<212>  DNA
<213>  Mus musculus

<400>  8
ggatttgggg aagcagaaat                                                  20


<210>  9
<211>  24
<212>  DNA
<213>  Mus musculus

<400>  9
aagagactaa aactgaggag cagc                                             24


<210>  10
<211>  17
<212>  DNA
<213>  Mus musculus

<400>  10
cgacagcaga gcgtgta                                                     17


<210>  11
<211>  18
<212>  DNA
<213>  Mus musculus

<400>  11
gacaatgctc tcacagac                                                    18


<210>  12
<211>  22
<212>  DNA
<213>  Mus musculus

<400>  12
ggagtggatt ctattattat gg                                               22


<210>  13
<211>  21
<212>  DNA
<213>  Mus musculus

<400>  13
gagaccgaag caccgactat g                                                21


<210>  14
<211>  21
<212>  DNA
<213>  Mus musculus

<400>  14
cggttttgac attgtgttcg c                                                21

<210>  15
<211>  21
<212>  DNA
<213>  Mus musculus

<400>  15
ggagtggaac agctgcgacc g                                                21

<210>  16
<211>  20
<212>  DNA
<213>  Mus musculus

<400>  16
gcacactcaa ccagcgtgcg                                                  20

<210>  17
<211>  22
<212>  DNA
<213>  Mus musculus

<400>  17
tgggcagaag gtgggactgt gt                                               22

<210>  18
<211>  20
<212>  DNA
<213>  Mus musculus

<400>  18
cgggaaaggc ggtatccggc                                                  20